# EUROPEAN PATENT APPLICATION

(11) **EP 2 438 909 A1**
(43) Date of publication of application: **11.04.2012**
(21) Application number: 10782982.2
(22) Date of filing: 03.06.2010
(51) Int. Cl.: A61K 9/107

(54) **PREPARATION METHOD OF DRUG LOADED EMULSION**

(30) Priority: 04.06.2009 CN 200910052535
(71) Applicant: Jiangsu Hengrui Medicine Co., Ltd., Lianyungang, Jiangsu 222002 (CN); Shanghai Hengrui Pharmaceutical Co. Ltd., Minhang District Shanghai 200-245 (CN)
(72) Inventor: TONG, Xinyong, Shanghai 200245 (CN); WANG, Haifeng, Shanghai 200245 (CN); YU, Li, Shanghai 200245 (CN); CHEN, Liang, Shanghai 200245 (CN); SHI, Yuan, Shanghai 200245 (CN)
(74) Representative: Jansen, Cornelis Marinus
(86) International application number: PCT/CN2010/073500
(87) International publication number: WO 2010/139278

(57) **Abstract**

A preparation method of drug loaded emulsion is disclosed. The method comprises the steps of: preparing a non-self emulsifying O/W blank emulsion having no active ingredients; then, adding therapeutically effective amount of active ingredients to the O/W blank emulsion, adjusting pH to distribute the active ingredients through the membrane to obtain the desired emulsion.

## Description

### FIELD OF THE INVENTION

The present invention relates to a preparation method of non-self emulsifying oil-in-water drug-loaded emulsion. Specifically, the present invention relates to a preparation method of non-self emulsifying oil-in-water drug-loaded emulsion by adding a drug to a blank emulsion.

### BACKGROUND OF THE INVENTION

Emulsions are widely used in clinic. As a carrier for drug delivery in parenteral system, fat emulsion has been applied for more than 40 years with advantages of increasing drug stability, decreasing toxicity, delaying or controlling drug release profile and improving drug targeting. In recent years, based on the developed preparation method of fat emulsion, the studies of drug-loaded emulsion were concerned increasingly. Drug-loaded products can both have treatment effect and provide energy to patients, which is favorable to help the patients get well.

There are some preparation methods of drug-loaded emulsion by using self emulsifying technology in the prior art. For example, Xiujuan Li and Liwei Zhang prepared aspirin water-in-oil microemulsion by using self emulsifying technology (Chemical Research and Application, March 2008, vol. 20, no. 3, p 352-355). Being gently stirred, the self emulsifying emulsion can spontaneously form an emulsion with a particle size less than 1000 nm. Therefore, a drug can be added to the system when preparing water phase or oil phase, and easily encapsulated inside the inner phase of the emulsion. But during the preparation of self emulsifying emulsion, large amount of emulsifier is needed, and co-emulsifier, generally organic solvent, is necessarily added, which results in self emulsifying emulsion having more toxicity for injection.

To decrease the toxicity caused by emulsifier and co-emulsifier in self emulsifying emulsion, non-self emulsifying oil-in-water emulsion with particle size less than 1000 nm, which has good dynamic stability and vascular permeability, was prepared generally by using high-speed stirring, high pressure homogenizing or microfluidizer and so on, to overcome the strong interfacial tension between oil and water. At present, the common preparation method of non-self emulsifying drug-loaded emulsion includes the next steps: First, prepare and mix oil phase and water phase. Second, prepare the coarse emulsion; Third, decrease the particle size of the emulsion by adapting severe condition mentioned above to obtain the final emulsion. Usually drug incorporation occurs in the 1st or the 2nd step. The drug incorporation methods include such ways as follows: 1. Dissolve the drug into the oil phase firstly if the drug is oil soluble, and then make it to be an emulsion; 2. Dissolve the drug into water phase firstly if the drug is water soluble, and then make it to be an emulsion; 3. Dissolve the drug, oil phase or/and emulsifier into suitable organic solvent firstly if the drug is not soluble in both oil phase and water phase, and then remove the organic solvent and make it to be an emulsion.

Many prior arts have disclosed drug incorporation occurred in the first step of preparation of the oil phase and water phase, such as Chinese patent application publication No. CN1399959A, etomidate fat emulsion injection preparation method; CN1546016A, nanopreparation of natural vitamin E and its preparation method; CN1634021A, novel paclitaxel emulsion for intravenous injection and its preparation method; CN1679576A, stabilized oil-in-water emulsion of vinca alkaloids for vein and production thereof; CN1732936A, nimodipine emulsion injection and method for preparing the same; CN1947720A, clarithromycin lipid microsphere injection and its preparation method; CN1861085A, isopropylphenol phosphosphingolipid fat emulsion and its preparation method; and so on. But Chinese patent application publication No. CN1771954A, vinorelbine lipid microsphere injection and its preparation method, disclosed a method of incorporating drug in the second step of preparing the coarse emulsion.

According to the general preparation process of drug-loaded emulsion in the prior art, drug is usually incorporated in the first or the second step when the drug is soluble in water phase or oil phase. That is, drug is incorporated at the beginning of preparation process and exists in the system, on one hand, the drug stays in the system for a long time, on the other hand, drug and other substances in the system go through several times of homogenization together to obtain final emulsion. For some unstable drugs, there are more chances to be denatured, inactive, structure destroyed or degraded because of long retention time and several times of homogenization, in particular strong shear force, high temperature and pressure caused by the above homogenization condition in the preparation process. But if the drug is not soluble both in oil phase and water phase, organic solvent, other cosolvents or additives usually should be added into system to improve drug solubility. Thus organic solvent residues or formulation complexity will occur, and further bring about the safety problems of emulsion.

Chinese patent application publication No. CN1620283A disclosed a method of preparing slightly or poorly soluble active ingredients to dispersion. An active ingredient was triturated with commercial O/W emulsion to produce a disperse system. The disperse system, in which the active ingredient was found primarily in the water phase, simultaneously comprises oil drops and active ingredient crystals as an inner phase. Then the disperse system was subjected to high pressure homogenization (e.g. 1500 bar and 5-20 homogenization cycles). After homogenizing, the active ingredient was incorporated and dissolved into inner phase of oil drops. In this preparation method of drug-loaded emulsion, although the drug incorporation procedure occurs after obtaining blank O/W emulsion, which shortens the retention time of drug in the whole system, generally the drug should be comminuted till its particle size less than 1000 nm before it was incorporated into the blank O/W emulsion. But during the incorporation process, a higher energetic homogenization was needed, resulting in powerful machine energy when the drug dissolved into the oil phase of emulsion. Thus this method is unsuitable to some unstable drugs. On the other hand, though this method avoids using organic solvent for solubilization, it puts forward a high requirement on emulsion preparation equipment, which has large energy consumption.

Phase transformation temperature method was used to prepare 10-Hydroxycamptothecin nano-emulsions by Gong Mingtao, Zhang Junshou et al (Chinese Journal of Natural Medicines, 2005, vol. 3, no. 1, p41-43). In this method, the drug was incorporated into blank O/W emulsion pre-prepared, but the drug was encapsulated into the inner phase of the emulsion through phase transformation, when the temperature was above 70 °C (a phase transformation temperature). The drug incorporation is conducted above a phase transformation temperature, so it is limited to some thermally unstable drugs.

### DESCRIPTION OF THE INVENTION

Drug instability and insolubility will reduce drug-loaded emulsion preparation quality, especially when the said drug is chemically unstable, or insoluble in oil phase or/and water phase, and particularly the said emulsion average particle size is less than 1000 nm. To overcome the deficiency in the prior art of drug-loaded emulsion preparation, the present invention introduce a drug self-loaded technology to prepare non-self emulsifying emulsion with drug loaded efficiently under gentle condition, particularly non-self emulsifying emulsion with particle size less than 1000 nm. The method comprises the following steps of:
preparing non-self emulsifying oil-in-water blank emulsion having no drug, preferably the particle size of the non-self emulsifying oil-in-water blank emulsion being less than 1000 nm;
adding therapeutically amount of drug into the blank emulsion, adjusting pH value, and transferring the drug across the membrane into the oil droplets of the emulsion by mixing to obtain a drug-loaded emulsion, preferably adjusting pH value to increase the oil-water partition coefficient of the drug.

According to the method of the present invention, firstly non-self emulsifying oil-in-water blank emulsion having no drug is prepared by using routine method, in which the particle size of the emulsion should satisfy clinical need, for example, the particle size is less than 1000 nm. Then the drug is added to the blank emulsion, as well as dissolved or dispersed into the outer water phase of the blank emulsion. Finally the pH value of the outer phase is adjusted to transform the drug in the outer phase to be more lipophilic such as in the form of free molecules, sequentially increase the oil-water partition coefficient 1gP of the drug. Under concentration difference, the drug is spontaneously transferred from the outer water phase into the inner oil phase of oil-in-water emulsion with the results that drug self loaded procedures can be achieved gently.

The non-self emulsifying oil-in-water blank emulsion according to the present invention refers to non-self emulsifying oil-in-water emulsion without therapeutically amount of the drug, which is equal to the blank final emulsion according to the prior art, and its particle size is as same as that of the final drug-loaded emulsion. It means that the drug in the present invention is added after the blank final emulsion has been prepared, other than the drug is added to the oil phase, the water phase or the coarse emulsion at the beginning. Thus the retention time of the drug in the system can be shortened. Since the drug is added to the emulsion after the blank final emulsion has been prepared, the subsequent preparation process does not require severe conditions to reduce emulsion particle size. Therefore the damage to the structure and the stability of sensitive drug molecules, which caused by strong shear force, high temperature and high pressure emerging from severe conditions such as stirring with high speed and homogenization under high pressure, can be avoided effectively.

Furthermore, according to the present invention, drug is added to the blank final emulsion, then the pH value of the outer phase is adjusted to transform the drug in the outer phase to be more lipophilic such as in the form of free molecules, sequentially increase the oil-water partition coefficient 1gP of the drug, thus the said concentration difference emerged between the inner phase and outer phase of the emulsion. Under concentration difference, the drug is spontaneously transferred from the outer water phase into the inner oil phase of oil-in-water emulsion. So these procedures reduce the needed machine energy which promote drug dissolution and help drug to be encapsulated into the inner phase. On the one hand, drug encapsulation efficiency can be improved. On the other hand, drug can be carried into the inner oil phase of O/W emulsion by a gentle mixture method.

Several preparation methods in the prior art can be used to prepare the non-self emulsifying oil-in-water blank emulsion according to the present invention, such as mechanical method, alternate addition method, phase transformation temperature method, phase transformation method, and so on. Taking the mechanical method as example, the preparation method of the non-self emulsifying oil-in-water blank emulsion comprises the following steps of: (1) preparing the oil phase; (2) preparing the water phase; (3) mixing the oil phase and the water phase, dispersing them uniformly, then preparing oil-in-water blank emulsion by using emulsifying machine. The emulsifying machine may be mortar, stir machine, colloid grinder, ultrasonic emulsification device, high pressure homogenizer, or microfluidizer and so on.

The drug according to the present invention can be water soluble, oil soluble, lightly soluble, slightly soluble or very slightly soluble in oil phase and/or water phase. Here lightly soluble means 1 g (or ml) solute can be dissolved into 30-100 ml solvent; slightly soluble means 1 g (or ml) solute can be dissolved into 100-1000 ml solvent; very slightly soluble means 1 g (or ml) solute can be dissolved into 1000-10000 ml solvent. Oil soluble drug may exist at a dissolved state in the O/W emulsion. It will be encapsulated by the oil drops to obtain an emulsion formed with oil drops heterogeneously dispersed. Water soluble drug can be transformed to be oil soluble drug by adjusting pH value in the outer phase. Then the drug can cross the membrane by gentle stirring. And also it will be encapsulated by the oil drops to obtain an emulsion formed with oil drops a heterogeneously dispersed. For drugs lightly soluble, slightly soluble or very slightly soluble, they are partially dissolved in the oil phase, and partially present in the emulsion in the form of nano crystalline highly dispersed to obtain an emulsion formed with oil drops heterogeneously dispersed and drug crystals together. The drug according to the present invention, whatever which is oil soluble, water soluble, lightly soluble, slightly soluble or very slightly soluble in oil phase and/or water phase, is preferably drugs with a solubility varying with the pH value.

The drug according to the present invention refers to an active ingredient for treating diseases of human or animal, which is selected from the group consisting of antitumor drugs, cardiovascular drugs, antiinfectives, antimycotics, virustatics, antiallergics, antiinflammytory drugs, endocrine agents, psychotics drugs, antibiotics, immunosupressives, vitamins and narcotic. Specially, the drug may be paclitaxel, docetaxel, vinorelbine, vincristine, hydroxycamptothecine, oxaliplatin, Lipo PGE, nimodipine, ciclosporin, itraconazole, amphotericin, acyclovir, dexamethasone, dexamethasone palmitate, indometacin, diazepam, clarithromycin, pingyangmycin, doxorubicin, vitamin A, vitamin D₂, vitamin E, vitamin K, bupivacaine, propofol, etomidate, Flurbiprofen Axetil, and so on.

The said drug can be added into the blank emulsion in the form of powder, solution or dispersion. First, the drug is added into the blank emulsion. Then the pH value of the outer water phase is adjusted, the said emulsion is stirred uniformly, and water is added to the final volume. After filtering, filling, sealing and sterilizing, the invented product is obtained.

As mentioned above, the present mixing is not directed to reduce the emulsion particle size, but acts as an auxiliary mechanical method to transform the drug into oil inner phase under concentration difference. The general mixing ways used in prior art can be applied in the present invention, such as mechanical stirring, high speed shearing, ultrasonic emulsifying, high pressure homogenizing or microfluidizer, preferably mechanical stirring, high speed shearing. Since pH value of the blank emulsion system added with drug is adjusted in the present preparation method, the oil-water partition coefficient is improved. Under concentration difference, the drug is spontaneously encapsulated into the inner oil phase, and more readily dispersed in the oil phase. Therefore, the present mixing does not need high energy or conditions with higher energy, which acts as an auxiliary mechanical method to transform the drug into oil inner phase under concentration difference. Even some mixing ways with severe conditions are adopted, such as ultrasonic emulsifying, high pressure homogenizing, microfluidizer, and so on, some conditions can be controlled. For example, the ultrasonic frequency can be reduced and preparation time can be shorten in the method of ultrasonic emulsifying, the pressure and cycling times can be controlled in the method of high pressure homogenizing. These measures can reduce the mixting energy, so that help the drug entering into the oil inner phase, meanwhile avoid negative influences on the drug.

Sterilization method can be selected from the group consisting of autoclaving, aseptic filtration, and other methods used for general emulsion sterilization in the prior art.

The drug-loaded emulsion according to the present invention comprises drug, solvent oil, surfactant and water. According to different physical and chemical properties of drugs, the drug-loaded emulsion can further comprise one or more components selected from the group consisting of stabilizer, solubilizer, cosolvent, metal chelator, osmotic pressure regulator, antioxidant and preservative.

The solvent oil according to the present invention includes one or more mineral oil, plant oil, animal oil or synthetic oil. The said plant oil is selected from the group consisting of soybean oil, safflower oil, corn oil, coconut oil, castor oil, brucea javanica oil, palm oil, medium chain triglycerides, peanut oil, cottonseed oil and a mixture thereof. The said animal oil is selected from the group consisting of fish oil, sperm oil and a mixture thereof. The suitable solvent oil can be selected according to the administration way of the drug loaded and emulsion thereof. The said solvent oil is presented in the emulsion in the range of 2-40 w/v%.

The surfactants according to the present invention include phospholipids, nonionic surfactant and a mixture thereof, which can be dissolved into the oil phase or dispersed into the water phase. The said phospholipids according to the present invention are selected from the group consisting of egg lecithin, soybean lecithin, hydrogenated egg lecithin, hydrogenated soybean phosphatidylcholine and synthetic phospholipid. The said nonionic surfactant is selected from the group consisting of Tween 20, Tween 40, Tween 60, Tween 80, Tween 85, Span 20, Span 40, Span 60, Span 80, polyoxyethylene castor oil, poly (ethylene oxide) hydrogen castor oil, poly (ethylene oxide) stearic acid ester, poloxamer188, polyethylene glycol stearate 15, polyethylene glycol- vitamin E succinate and a mixture thereof. The said surfactant is presented in the emulsion in the range of 0.5-50 w/v %.

The antioxidant according to the present invention can be selected from the group consisting of water-soluble antioxidant and oil-soluble antioxidant. Water-soluble antioxidant is dissolved in the water phase and oil-soluble antioxidant is dissolved in the oil phase. The water-soluble antioxidant is selected from the group consisting of sodium sulfite, sodium hydrogensulfite, sodium metabisulfite, ascorbic acid, sodium ascorbate, L-cysteine and a mixture thereof. The said oil-soluble antioxidant is selected from the group consisting of α-tocopherol, α-tocopheryl acetate, α-tocopherol succinate, butyl hydroxy anisole (BHA), Butylated hydroxytoluene (BHT) and a mixture thereof.

The metal chelator according to the present invention is selected from the group consisting of EDTA, EDTA disodium salt, EDTA dicalcium salt and a mixture thereof.

The osmotic pressure regulator according to the present invention is selected from the group consisting of glycerin, sorbitol, mannitol, glucose, sodium chloride and a mixture thereof.

The stabilizer according to the present invention is selected from the group consisting of oleic acid, sodium oleate, cholesterol, cholic acid, sodium cholate, deoxycholic acid, deoxysodium cholate and a mixture thereof.

The preservative according to the present invention is selected from the group consisting of clove oil, propylene glycol, sorbitol, sorbic acid, methane acid, calclum butylparaben, sodium methylparaben, sodium propylparaben, benzyl alcohol, benzoic acid and a mixture thereof.

The cosolvent according to the present invention is selected from the group consisting of ethyl oleate, benzyl benzoate, benzyl alcohol, ethyl lactate, ethanol, 1,2-propylene glycol, polyethylene glycol and a mixture thereof.

The drug-loaded emulsion according to the present invention can be administrated locally, orally or parenterally, especially intravenously, endermicly, subcutaneously, intramuscularly, intraarticular or intrapleurally, preferably intravenously. For intravenously administration, the average particle size of the drug-loaded emulsion is less than 1000 nm.

Comparing with the prior art, the present invention have advantages as follows:
1. According to the present invention, the oil-water partition coefficient lgP of the drug is changed by adjusting pH value, and the drug is redistributed between the oil phase and water phase of the blank emulsion, thereby drug is transported across the membrane and is distributed into the oil phase in a gently way. Thus organic solvent will not be needed. And the resulting product will not have the problem of solvents residual.
2. Comparing with the general preparation methods of drug-loaded emulsion, the drug according to the present invention is added after the preparation of the blank final O/W emulsion, thus the retention time of the drug in the system is reduced. In addition, no mechanical stirring or homogenizing with high pressure or energy will be taken in or after the drug adding procedure, which could reduce or avoid drug degradation during the preparation process.
3. The preparation method according to the present invention is simple and convenient, needs no special emulsion preparation devices, such as emulsification devices producing high energy. It is favorable to mass industrial manufacture of the formulation.

### PREFERRED EMBODIMENTS

The following examples are intended to illustrate the invention, but are in no way intended to limit the scope thereof.

### EXAMPLE1

| | |
|---|---|
| vinorelbine tartrate | 0.05 % |
| soybean oil | 5 % |
| egg lecithin | 2 % |
| Water for injection | up to 1000 ml |

Under the protection of inert gas, 50 g soybean oil was taken and preheated to 75 °C; and 20 g egg lecithin was added to appropriate amount of water for injection, then the liquid was stirred uniformly to obtain the water phase, and the water phase was preheated to 75 C. Under high-speed stirring, the water phase was added into the oil phase, and the liquid was homogenized uniformly with high pressure homogenizer to obtain blank O/W emulsion with particle size less than 1000 nm. The emulsion was added with 0.5 g Vinorelbine Tartrate, adjusted pH value to 8.0, stirred mechanically, then added with Water for injection to the constant volume of 1000 ml. The emulsion was sterilized by filtration with 0.22 µm filter, then filled under the protection of nitrogen, and the container was sealed.

### EXAMPLE 2

| | |
|---|---|
| bupivacaine HCl | 0.1 % |
| castor oil | 10 % |
| soybean lecithin | 2 % |
| anhydrous sodium sulfite | 0.2 % |
| glycerin | 2.5 % |
| Water for injection | up to 1000 ml |

Under the protection of inert gas, 100 g castor oil was taken and preheated to 60 °C; and 20 g soybean lecithin, 2 g anhydrous sodium sulfite, 25 g glycerin were added to appropriate amount of water for injection, then the liquid was stirred uniformly to obtain the water phase, and the water phase was preheated to 60 °C. Under high-speed stirring, the oil phase and the water phase were homogeneously mixed and homogenized with high pressure homogenizer to obtain blank O/W emulsion with particle size less than 1000 nm. The emulsion was added with 1 g bupivacaine HCl, adjusted pH to 7.0, stirred mechanically, then added with water for injection to the constant volume of 1000 ml. The emulsion was sterilized by filtration with 0.22 µm filter, then filled under the protection of nitrogen, and the container was sealed.

### EXAMPLE 3

The formulation here is as same as the example 2, except that pH value was adjusted to 4.0 during the process.

### EXAMPLE 4

The formulation here is as same as the example 2, except that pH value was adjusted to 5.0 during the process.

### EXAMPLE 5

The formulation here is as same as the example 2, except that pH value was adjusted to 6.0 during the process.

### EXAMPLE 6

The formulation here is as same as the example 2, except that pH value was adjusted to 8.0 during the process.

Content, related substance, encapsulation efficiency and particle size of examples 2-6 were determined, and the results were shown in tables 1 and 2:

**Table 1 Determination results before accelerating test**

| | Particle size(nm) | content(%) | encapsulation efficiency(%) | Related substance(%) |
|---|---|---|---|---|
| Example 2 (pH 7.0) | 130.4 | 99.6 | 86.9 | 0.42 |
| Example 3 (pH 4.0) | 132.6 | 98.6 | 35.8 | 0.45 |
| Example 4 (pH 5.0) | 139.5 | 99.4 | 52.9 | 0.48 |
| Example 5 (pH 6.0) | 130.7 | 98.8 | 70.2 | 0.47 |
| Example 6 (pH 8.0) | 129.8 | 99.1 | 96.7 | 0.58 |

**Table 2 Determination results of accelerating test for one month (25°C)**

| | Particle size(nm) | content(%) | encapsulation efficiency (%) | Related substance(%) |
|---|---|---|---|---|
| Example 2 (pH 7.0) | 131.2 | 98.9 | 87.2 | 0.72 |
| Example 3 (pH 4.0) | 132.4 | 99.0 | 36.4 | 0.48 |
| Example 4 (pH 5.0) | 139.2 | 98.8 | 51.8 | 0.54 |
| Example 5 (pH 6.0) | 130.2 | 98.4 | 69.5 | 0.62 |
| Example 6 (pH 8.0) | 129.5 | 98.6 | 95.9 | 0.82 |

It is shown from examples 2-6 that the pH value of emulsion thereby influences the encapsulation efficiency obviously by changing the oil-water partition coefficient of bupivacaine HCl. The lower the pH value is, the lower the oil-water partition coefficient of drug will be. A low pH value cause small amount of drug entering into the inner phase of the emulsion and low encapsulation efficiency. As the rising of the pH value, the encapsulation efficiency increase obviously.

Mean particle size, content and related substances of different formulation had different changes after accelerating test for one month. The possible reason was that bupivacaine hydrochloride was in the form of water soluble hydrochloride salt when the pH value was low. Lower pH value, Lower oil-water partition coefficient. So it was more stable than the drug in the form of lipid soluble free base, and the related substances was less than that of the formulation with higher pH value. pH value had no significant effect on the content and particle size.

### EXAMPLE 7

| | |
|---|---|
| irinotecan hydrochloride | 0.05 % |
| soybean oil | 10 % |
| egg lecithin | 2 % |
| poloxamer | 2 % |
| glycerin | 2.5 % |
| Water for injection | up to 1000 ml |

Under the protection of inert gas, 100 g soybean oil was taken as the oil phase; and 20 g egg lecithin, 20 g poloxamer, 25 g glycerin were added to appropriate amount of water for injection, then the liquid was stirred uniformly to obtain the water phase. Under high-speed stirring, the oil phase and the water phase were homogeneously mixed and homogenized with high pressure homogenizer to obtain blank O/W emulsion with particle size less than 1000 nm. The emulsion was added with 0.5 g irinotecan hydrochloride, adjusted pH value to 8.0, emulsified with micro jet stream homogenizer, then added with water for injection to the constant volume of 1000 ml. The emulsion was sterilized by rotary, then filled under the protection of nitrogen, and the container was sealed.

### EXAMPLE 8

| | |
|---|---|
| vinorelbine | 0.2 % |
| soybean oil | 5 % |
| egg lecithin | 2 % |
| Water for injection | up to 1000 ml |

Under the protection of inert gas, 50 g soybean oil was taken and preheated to 75 °C; and 20 g egg lecithin was added to appropriate amount of water for injection, then the liquid was stirred uniformly to obtain the water phase, and the water phase was preheated to 75 °C. Under high-speed stirring, the water phase was added into the oil phase, and the liquid was homogenized uniformly with high pressure homogenizer to obtain blank O/W emulsion with particle size less than 1000 nm. The emulsion was added with 2 g vinorelbine, adjusted pH value to 8.0, stirred mechanically, then added with water for injection to the constant volume of 1000 ml. The emulsion was sterilized by filtration with 0.22 µm filter, then filled under the protection of nitrogen, and the container was sealed.

### EXAMPLE 9

| | |
|---|---|
| Docetaxel | 0.05 % |
| medium chain oil | 15 % |
| egg lecithin | 2 % |
| Tween 80 | 0.5 % |
| oleic acid | 0.4 % |
| glycerin | 2.5 % |
| VE | 0.05 % |
| Water for injection | up to 1000 ml |

Under the protection of inert gas, 150 g medium chain oil, 4 g oleic acid, 0.5 g VE and 5 g Tween-80 were stirred uniformly to obtain the oil phase, and the oil phase was preheated to 70 C; 20 g egg lecithin and 25 g glycerin were added to appropriate amount of water for injection, then the liquid was stirred uniformly to obtain the water phase, and the water phase was preheated to 70 °C. Under high-speed stirring, the oil phase and the water phase were homogeneously mixed and homogenized with high pressure homogenizer to obtain blank O/W emulsion with particle size less than 1000 nm. The emulsion was added with 0. 5g Docetaxel, adjusted pH value to 5.0, emulsified with micro jet stream homogenizer, then added with water for injection to the constant volume of 1000 ml. The emulsion was sterilized by rotary, then filled under the protection of nitrogen, and the container was sealed.

### EXAMPLE 10

| | |
|---|---|
| Amphotericin B | 0.2 % |
| fish oil | 10 % |
| egg lecithin | 2 % |
| sodium oleate | 0.1 % |
| glycerin | 2.5 % |
| EDTA disodium salt | 0.01 % |
| Water for injection | up to 1000 ml |

Under the protection of inert gas, 100 g fish oil was taken and preheated to 70 C; and 20 g egg lecithin, 1 g sodium oleate, 0.1 g EDTA disodium salt, 25 g glycerin were added to appropriate amount of water for injection, then the liquid was stirred uniformly to obtain the water phase, and the water phase was preheated to 70 C. Under high-speed stirring, the oil phase and the water phase were homogeneously mixed and homogenized with high pressure homogenizer to obtain blank O/W emulsion. The emulsion was added with 2 g Amphotericin B, adjusted pH value to 7.0, emulsified with high pressure homogenizer till particle size less than 1000 nm, then added with water for injection to the constant volume of 1000 ml. The emulsion was sterilized by rotary, then filled under the protection of nitrogen, and the container was sealed.

### EXAMPLE 11

| | |
|---|---|
| Lipo PGE | 0.001 % |
| soybean oil | 10 % |
| egg lecithin | 1.2 % |
| oleic acid | 0.1 % |
| glycerin | 2.5 % |
| Water for injection | up to 1000 ml |

Under the protection of inert gas, 100 g soybean oil, 1 g oleic acid were stirred uniformly to obtain the oil phase; 12 g egg lecithin and 25 g glycerin were added to appropriate amount of water for injection, then the liquid was stirred uniformly to obtain the water phase. Under high-speed stirring, the oil phase and the water phase were homogeneously mixed and homogenized with high pressure homogenizer to obtain blank O/W emulsion with particle size less than 1000 nm. The emulsion was added with 10 mg Lipo PGE, adjusted pH value to 5.0, emulsified with high pressure homogenizer, then added with water for injection to the constant volume of 1000 ml. The emulsion was sterilized by rotary, then filled under the protection of nitrogen, and the container was sealed.

### EXAMPLE 12

| | |
|---|---|
| diazepam | 0.1 % |
| soybean oil | 10 % |
| egg lecithin | 1.2 % |
| sodium oleate | 0.05 % |
| glycerin | 2.5 % |
| Water for injection | up to 1000 ml |

Under the protection of inert gas, 100 g soybean oil was taken as the oil phase; 12 g egg lecithin, 0.5 g sodium oleate and 25 g glycerin were added to appropriate amount of water for injection, then the liquid was stirred uniformly to obtain the water phase. Under high-speed stirring, the oil phase and the water phase were homogeneously mixed and homogenized with high pressure homogenizer to obtain blank O/W emulsion with particle size less than 1000 nm. The emulsion was added with 1 g diazepam, adjusted pH value to 8.0, emulsified with high pressure homogenizer, then added with water for injection to the constant volume of 1000 ml. The emulsion was sterilized by rotary, then filled under the protection of nitrogen, and the container was sealed.

### EXAMPLE 13

| | |
|---|---|
| etomidate | 0.1 % |
| soybean oil | 10 % |
| egg lecithin | 1.2 % |
| glycerin | 2.5 % |
| Water for injection | up to 1000 ml |

Under the protection of inert gas, 100 g soybean oil was taken and preheated to 50 C; and 12 g egg lecithin, 25 g glycerin were added to appropriate amount of water for injection, then the liquid was stirred uniformly to obtain the water phase, and the water phase was preheated to 50 C. Under high-speed stirring, the oil phase and the water phase were homogeneously mixed and homogenized with high pressure homogenizer to obtainblank O/W emulsion with particle size less than 1000 nm. The emulsion was added with 1 g etomidate, adjusted pH value to 5.0, emulsified with high pressure homogenizer, then added with water for injection to the constant volume of 1000 ml. The emulsion was sterilized by rotary, then filled under the protection of nitrogen, and the container was sealed.

### EXAMPLE 14

| | |
|---|---|
| propofol | 0.1 % |
| soybean oil | 5 % |
| medium chain oil | 5 % |
| egg lecithin | 1.2 % |
| glycerin | 2.5 % |
| Water for injection | up to 1000 ml |

Under the protection of inert gas, 50 g soybean oil, 50 g medium chain oil were stirred uniformly to obtain the oil phase, and the oil phase was preheated to 60 C; 12 g egg lecithin and 25 g glycerin were added to appropriate amount of water for injection, then the liquid was stirred uniformly to obtain the water phase, and the water phase was preheated to 60 C. Under high-speed stirring, the oil phase and the water phase were homogeneously mixed and homogenized with high pressure homogenizer to obtain blank O/W emulsion with particle size less than 1000 nm. The emulsion was added with 1 g propofol, adjusted pH value to 8.0, emulsified with high pressure homogenizer, then added with water for injection to the constant volume of 1000 ml. The emulsion was sterilized by rotary, then filled under the protection of nitrogen, and the container was sealed.

### EXAMPLE 15

| | |
|---|---|
| ciclosporin | 0.1 % |
| medium chain oil | 3 % |
| polyoxyethylene castor oil | 10 % |
| PEG-400 | 5 % |
| NaCl | 0.4 % |
| Water for injection | up to 1000 ml |

Under the protection of inert gas, 30 g medium chain oil was taken and preheated to 60 C; 100 g polyoxyethylene castor oil, 50 g PEG-400 and 4 g NaCl were added to appropriate amount of water for injection, then the liquid was stirred uniformly to obtain the water phase, and the water phase was preheated to 60 C. Under high-speed stirring, the oil phase and the water phase were homogeneously mixed and homogenized with high pressure homogenizer to obtain blank O/W emulsion with particle size less than 1000 nm. The emulsion was added with 1g ciclosporin, adjusted pH value to 7.4, emulsified with high speed shearing, then added with water for injection to the constant volume of 1000 ml. The emulsion was sterilized by filtration with 0.22 µm filter, then filled under the protection of nitrogen, and the container was sealed.

### EXAMPLE 16

| | |
|---|---|
| nimodipine | 0.1 % |
| soybean oil | 15 % |
| soybean lecithin | 1.2 % |
| sodium oleate | 0.05 % |
| glycerin | 2.25 % |
| Water for injection | up to 1000 ml |

Under the protection of inert gas, 150 g soybean oil, 12 g soybean lecithin were stirred uniformly to obtain the oil phase, and the oil phase was preheated to 60 C; 22.5 g glycerin and 0.5 g sodium oleate were added to appropriate amount of water for injection, then the liquid was stirred uniformly to obtain the water phase, and the water phase was preheated to 60 C. Under high-speed stirring, the oil phase and the water phase were homogeneously mixed and homogenized with high pressure homogenizer to obtain blank O/W emulsion with particle size less than 1000 nm. The emulsion was added with 1 g nimodipine, adjusted pH value to 8.0, emulsified with high pressure homogenizer, then added with water for injection to the constant volume of 1000 ml. The emulsion was sterilized by rotary, then filled under the protection of nitrogen, and the container was sealed.

### EXAMPLE 17

| | |
|---|---|
| vitamin D₂ | 0.5 % |
| soybean oil | 5 % |
| soybean lecithin | 1.5 % |
| glycerin | 2.25 % |
| Water for injection | up to 1000 ml |

Under the protection of inert gas, 50 g soybean oil, 15 g soybean lecithin were stirred uniformly to obtain the oil phase, and the oil phase was preheated to 70 C; 22.5 g glycerin was added to appropriate amount of water for injection, then the liquid was stirred uniformly to obtain the water phase, and the water phase was preheated to 70 C. Under high-speed stirring, the oil phase and the water phase were homogeneously mixed and homogenized with high pressure homogenizer to obtain blank O/W emulsion with particle size less than 1000 nm. The emulsion was added with 5 g vitamin D₂, adjusted pH value to 8.0, emulsified with high pressure homogenizer, then added with water for injection to the constant volume of 1000 ml. The emulsion was sterilized by rotary, then filled under the protection of nitrogen, and the container was sealed.

### EXAMPLE 18

| | |
|---|---|
| acyclovir | 0.5 % |
| soybean oil | 10 % |
| soybean lecithin | 2 % |
| sodium oleate | 0.05 % |
| glycerin | 2.25 % |
| Water for injection | up to 1000 mL |

Under the protection of inert gas, 100 g soybean oil was taken and preheated to 70 C; 20 g soybean lecithin, 22.5 g glycerin and 0.5 g sodium oleate were added to appropriate amount of water for injection, then the liquid was stirred uniformly to obtain the water phase, and the water phase was preheated to 70 C. Under high-speed stirring, the oil phase and the water phase were homogeneously mixed and homogenized with high pressure homogenizer to obtain blank O/W emulsion with particle size less than 1000 nm. The emulsion was added with 5g acyclovir, adjusted pH to 8.0, emulsified with high pressure homogenizer, then added with water for injection to the constant volume of 1000 ml. The emulsion was sterilized by rotary, then filled under the protection of nitrogen, and the container was sealed.

### EXAMPLE 19

The formulation here is as same as the example 18, except that the drug is replaced by clarithromycin, and the amount is 0.1 % of the formulation.

### EXAMPLE 20

The formulation here is as same as the example 18, except that the drug is replaced by dexamethasone, and the amount is 0.1 % of the formulation.

### EXAMPLE 21

The formulation here is as same as the example 18, except that the drug is replaced by doxorubicin, and the amount is 0.1 % of the formulation.

## Claims

1. A method for preparing non-self emulsifying drug-loaded oil-in-water emulsion, **characterized in** comprising the following steps of:
preparing non-self emulsifying oil-in-water blank emulsion having no drug, preferably the particle size of the non-self emulsifying oil-in-water blank emulsion being less than 1000nm;
adding therapeutically amount of drug into the blank emulsion, adjusting pH value and transferring the drug across the membrane into the oil droplets of the emulsion by mixing to obtain a drug-loaded emulsion, preferably adjusting pH value to increase the oil-water partition coefficient of the drug.

2. The method according to claim 1, **characterized in that** the said drug is present in the emulsion at a dissolved state, and is encapsulated in the oil droplets to obtain an emulsion formed with oil drops heterogeneously dispersed; or the said drug is partially present in the emulsion in the form of nano crystalline highly dispersed, and partially dissolved in the oil phase to obtain an emulsion formed with oil drops heterogeneously dispersed and drug crystals together.

3. The method according to any one of claims 1 to 2, **characterized in that** the said drug is added to the blank emulsion in the form of powder, solution or dispersion.

4. The method according to any one of claims 1 to 3, **characterized in that** the said mixing is selected from the group consisting of mechanical stirring, high speed shearing, ultrasonic wave emulsifying, high pressure homogenizing and microfluidizer.

5. The method according to any one of claims 1 to 4, **characterized in that** the said drug-loaded emulsion comprises drug, solvent oil, surfactant and water.

6. The method according to claim 5, **characterized in that** the said solvent oil is selected from the group consisting of mineral oil, plant oil, animal oil and synthetic oil and a mixture thereof.

7. The method according to claim 6, **characterized in that** the said plant oil is selected from the group consisting of soybean oil, safflower oil, corn oil, coconut oil, castor oil, brucea javanica oil, palm oil, medium chain triglycerides, peanut oil, cottonseed oil and a mixture thereof; the said animal oil is selected from the group consisting of fish oil, sperm oil and a mixture thereof.

8. The method according to claim 5, **characterized in that** the said surfactant is selected from the group consisting of phospholipids, nonionic surfactant and a mixture thereof; and the said surfactant is dissolved into the oil phase or dispersed into the water phase.

9. The method according to claim 8, **characterized in that** the said phospholipids are selected from the group consisting of egg lecithin, soybean lecithin, hydrogenated egg lecithin, hydrogenated soybean phosphatidylcholine and synthetic phospholipids; and the said nonionic surfactant is selected from the group consisting of Tween 20, Tween 40, Tween 60, Tween 80, Tween 85, Span 20, Span 40, Span 60, Span 80, polyoxyethylene castor oil, poly (ethylene oxide) hydrogen castor oil, poly (ethylene oxide) stearic acid ester, poloxamer 188, polyethylene glycol stearate 15, polyethylene glycol- vitamin E succinate and a mixture thereof.

10. The method according to any one of claims 1 to 9, **characterized in that** the said drug-loaded emulsion further comprises one or more components selected from the group consisting of stabilizer, solubilizer, cosolvent, metal chelator, osmotic pressure regulator, antioxidant and preservative.

11. The method according to claim 10, **characterized in that** the said antioxidant is selected from the group consisting of water-soluble antioxidant and oil-soluble antioxidant, water-soluble antioxidant being dissolved in the water phase and oil-soluble antioxidant being dissolved in the oil phase, wherein the said water-soluble antioxidant is selected from the group consisting of sodium sulfite, sodium hydrogensulfite, sodium metabisulfite, ascorbic acid, sodium ascorbate, L-cysteine and a mixture thereof; wherein the said oil-soluble antioxidant is selected from the group consisting of α-tocopherol, α-tocopheryl acetate, α-tocopherol succinate, butyl hydroxy anisole, butylated hydroxytoluene and a mixture thereof.

12. The method according to claim 10, **characterized in that** the said metal chelator is selected from the group consisting of EDTA, EDTA disodium salt, EDTA dicalcium salt and a mixture thereof.

13. The method according to claim 10, **characterized in that** the said osmotic pressure regulator is selected from the group consisting of glycerin, sorbitol, mannitol, glucose, sodium chloride and a mixture thereof.

14. The method according to claim 10, **characterized in that** the said stabilizer is selected from the group consisting of oleic acid, sodium oleate, cholesterol, cholic acid, sodium cholate, deoxycholic acid, deoxysodium cholate and a mixture thereof.

15. The method according to claim 10, **characterized in that** the said preservative is selected from the group consisting of clove oil, propylene glycol, sorbitol, sorbic acid, methane acid, calclum butylparaben, sodium methylparaben, sodium propylparaben, benzyl alcohol, benzoic acid and a mixture thereof.

16. The method according to claim 10, **characterized in that** the said cosolvent is selected from the group consisting of ethyl oleate, benzyl benzoate, benzyl alcohol, ethyl lactate, ethanol, 1,2-propylene glycol, polyethylene glycol and a mixture thereof.

17. The method according to any one of claims 1 to 16, **characterized in that** the said drug refers to an active ingredient used to treat diseases of human or animal, preferably the said drug is selected from the group consisting of antitumor drugs, cardiovascular drugs, antiinfectives, antimycotics, virustatics, antiallergics, antiinflammytory drugs, endocrine agents, psychotic drugs, antibiotics, immunosupressives, vitamins and narcotic.

18. The method according to any one of claims 1 to 17, **characterized in that** the said drug-loaded emulsion is in the form of local, oral or parenteral dosage form, preferably in the form of intravenous, endermic, subcutaneous, intramuscular, intraarticular or intrapleural dosage form, more preferably in the form of intravenous dosage form.

19. The method according to claim 18, **characterized in that** when the said drug-loaded emulsion is administrated intravenously, the average particle size of oil drops of the emulsion is less than 1000nm.
